# EUROPÄISCHE PATENTSCHRIFT

(11) **EP 1 235 513 B1**
(45) Veröffentlichungstag und Bekanntmachung des Hinweises auf die Patenterteilung: **07.09.2005**
(21) Anmeldenummer: 00964142.4
(22) Anmeldetag: 08.09.2000
(51) Int. Cl.: A61B 5/05

(54) **DIAGNOSTIZIEREINRICHTUNG**
DIAGNOSTIC DEVICE
DISPOSITIF DE DIAGNOSTIC

(30) Priorität: 11.12.1999 DE 19959808
(43) Veröffentlichungstag der Anmeldung: 04.09.2002
(73) Patentinhaber: Grösser, Hans Karl, 63450 Hanau (DE)
(72) Erfinder: Grösser, Hans Karl, 63450 Hanau (DE)
(74) Vertreter: Aue, Hans-Peter
(86) Internationale Anmeldenummer: PCT/EP2000/008777
(87) Internationale Veröffentlichungsnummer: WO 2001/041637

(56) Entgegenhaltungen:
- DE-A- 3 038 325
- DE-A- 19 717 337

## Beschreibung

Die Erfindung bezieht sich auf eine Diagnostiziereinrichtung zum Testen von Organen des Menschen durch Messung der Stromfrequenz des zu testenden Organs.

Es ist ein Diagnostizierverfahren mittels Stromfrequenzmessung zum Testen von Organen des Menschen bekannt, das insbesondere von Homöopathen und Heilpraktikern verwendet wird. Dabei wird eine Messelektrode an bestimmten Punkten von Meridianen links bzw. rechts an einem Finger der Hand einer Testperson angelegt und mittels Ultraschall die an den Messpunkten erzeugte Frequenz in Hertz gemessen.

Die Messpunkte sind empirisch gefundene Reaktionspunkte der Haut und tiefer gelegener Gewebeschichten und bilden den Ort der Reizübertragung auf lokale segment-reflektorische, vegetativ-reflektorische und neuro-endokrine Bereiche. Charakteristisch für eine Vielzahl von Messpunkten ist ihre lineare Anordnung als Punktkette, so genannte Meridiane. Diese Punktketten liegen im Grenzbereich zweier Segmente bevorzugt im Meridianverlauf, z.B. an den Extremitäten, wo eine Übereinander-Projektion von Dermatom, Myotom und Sklerotom am deutlichsten erkennbar wird. Diese Messpunkte lassen sich in ihrer topografischen Lage auf der Haut des Menschen durch Messung des Hautwiderstandes bzw. der Stromfrequenz ermitteln.

Am ermittelten Ergebnis der gemessenen Frequenz kann von der untersuchenden Person festgestellt werden, ob die Frequenz des mit diesem Verfahren untersuchten Organs der Testperson vom an sich bekannten Frequenzbereich des Organs abweicht oder nicht. Liegt die ermittelte Ultraschallfrequenz innerhalb des bekannten Frequenzbereiches des Organs, kann in der Regel davon ausgegangen werden, dass dieses Organ gesund ist. Weicht hingegen die gemessene Frequenz vom bekannten Frequenzbereich des zu untersuchenden Organs der Testperson ab, deutet dies auf eine Erkrankung des untersuchten Organs hin.

Weiterhin werden zum Testen von Organen des Menschen eine Vielzahl von Testsubstanzen verwendet, die beim Anlegen eines Stroms als Messergebnis ebenfalls eine bestimmte Frequenzinformation liefern. Um ein genaueres oder aussagekräftigeres Untersuchungsergebnis des zu untersuchenden Organs zu bekommen, wird das Ergebnis der Frequenzmessung der Testsubstanz mit dem Ergebnis der Frequenzmessung am Körper der Testperson verglichen und daraufhin auf den Zustand des zu untersuchenden Organs geschlossen. Bei der Messung der Testsubstanzen werden diese in Teströhrchen gefüllt und dem Messverfahren unterzogen.

Leidet eine Testperson unter einer Erkrankung eines bestimmten Organs, beispielsweise an einer Erkrankung des Herzens, nimmt diese meistens ein oder mehrere von einem Arzt verschriebene Medikamente ein. Diese Medikamente können, wie die vorstehend erwähnten Testsubstanzen, daraufhin untersucht werden, ob diese zur Behandlung des betreffenden Organs der Testperson geeignet sind.

Dieses auf Frequenzmessung basierende Verfahren, das über die Punktlokalisation hinaus einen Rückschluss aus der Höhe der gemessenen Haut- bzw. Gewebefrequenz auf den energetischen Zustand der Punkte und Meridiane zieht, ist bedingt durch die große Variabilität des Hautwiderstandes und dessen Störgrößen meist ungenau oder schlecht reproduzierbar. Diese Störgrößen basieren beispielsweise auf der Feuchtigkeit der Haut, Fettigkeit der Hautoberfläche, Zustand des vegetativen Nervensystems der Testperson, Andruck der Messelektrodenspitze auf den Messpunkt usw. und können die Messergebnisse erheblich beeinflussen. So ergibt beispielsweise eine feuchte Haut eine andere Frequenz wieder als eine trockene Haut. Darüber hinaus können die Messergebnisse durch geringste Variationen, wie körperliche Bewegung, Einnahme von anregenden Mitteln, beispielsweise Kaffee oder Tee, Aufregung, Nahrungsaufnahme, Raumtemperatur, laute Geräusche usw. völlig verfälscht werden.

Ein weiterer Nachteil dieses Verfahrens besteht darin, dass permanent eine enorme Vielzahl von Testsubstanzen für die unterschiedlichen Organe und Medikamente sowie Teströhrchen zur Verfügung stehen müssen, die sehr kostenintensiv sind. Die Testsubstanzen haben meistens nur eine begrenzte Haltbarkeitsdauer und müssen beim Erreichen des Verfallsdatums entsorgt und neue Testsubstanzen beschafft werden.

Es ist Aufgabe der Erfindung, eine Diagnostiziereinrichtung der eingangs genannten Art zu schaffen, die kostengünstig hergestellt und dauerhaft betrieben werden kann und dabei zuverlässige Messergebnisse liefert, die zur Therapie einer Testperson genutzt werden.

Erfindungsgemäß wird die Aufgabe gelöst durch eine Diagnostiziereinrichtung zum Testen von Organen des Menschen durch Messung der Stromfrequenz des zu testenden Organs beinhaltend zwei parallel zueinander beabstandete, vertikal aufgerichtete plattenförmige Bauteile, die mit einem zwischen diesen einbringbaren Messfühler elektrisch verbunden sind, wobei eines der plattenförmigen Bauteile mit einem mit dem Körper einer Testperson in Kontakt bringbaren Messelement und das andere plattenförmige Bauteil mit einer rechnergesteuerten Auswerteeinheit elektrisch verbunden sind. Zweckmäßigerweise bestehen die plattenförmigen Bauteile aus einem elektrisch leitenden Material.

Das mit dem Körper einer Testperson in Kontakt bringbare Messelement liefert ein Ergebnis auf der Basis der Haut- bzw. Gewebefrequenz, welche auf eines der plattenförmige Bauteile übertragen wird. Weiterhin wird von der rechnergesteuerten Auswerteeinheit eine von einem Computerprogramm bekannte Frequenz bzw. ein Frequenzbereich, die vorbekannt sind, auf das andere plattenförmige Bauteil übertragen. Dadurch wird zwischen den beiden zueinander beabstandeten plattenförmigen Bauteilen ein elektromagnetisches Feld erzeugt. In Abhängigkeit davon, ob das vom Messelement, das an den Körper einer Testperson in Kontakt gebracht ist, gelieferte Messergebnis mit dem von der rechnergesteuerten Auswerteeinheit gelieferten Frequenzvorgabe übereinstimmt oder wesentlich davon abweicht, hat das elektromagnetische Feld zwischen den plattenförmigen Bauteilen eine unterschiedliche Spannungscharakteristik. Mit Hilfe des zwischen die plattenförmigen Bauteile einbringbaren Messfühlers erfolgt eine Feinstrommessung. Dabei wird eine vorhandene Differenz im elektrischen Feld zwischen den beiden plattenförmigen Bauteilen gemessen und an die rechnergesteuerte Auswerteeinheit übermittelt, um diese Information auszuwerten. Mittels eines speziellen Computerprogramms kann ermittelt werden, ob und inwieweit das zu untersuchende Organ der Testperson erkrankt ist.

Darüber hinaus können vom Computerprogramm therapeutischen Maßnahmen und/oder Möglichkeiten der Einnahme von geeigneten Medikamenten vorgeschlagen werden. Damit ist das mit der erfindungsgemäßen Diagnostiziereinrichtung auszuführende Messverfahren beliebig oft reproduzierbar. Darüber hinaus werden durch die Abgleichung der Messwerte mit den Vorgaben der rechnergesteuerten Auswerteeinheit sehr genaue Resultate erzielt. Da keine Testsubstanzen und Teströhrchen mehr bevorratet werden müssen und die Diagnostiziereinrichtung unbeschränkt oft einsetzbar ist, kann sie ohne größere Kosten betrieben werden.

Zur Erreichung einer stabilen Gesamtkonstruktion der erfindungsgemäßen Diagnostiziereinrichtung sind die plattenförmigen Bauteile auf einem Basisträger befestigt sind. Dieser Basisträger kann beispielsweise eine Grundplatte sein. Dadurch wird die Diagnostiziereinrichtung ortsbeweglich und kann auf einfache Weise transportiert werden. Des Weiteren wird gewährleistet, dass der Abstand der beiden plattenförmigen Bauteile zueinander stets gleich ist, um möglichst einheitliche Testbedingungen zu schaffen und genaue Messergebnisse zu erzielen.

Nach einer Weiterbildung der Erfindung ist der Messfühler nach Art einer Antenne ausgebildet und ist zur Stromfrequenzmessung zwischen den zwei plattenförmigen Bauteilen vorgesehen. Dabei kann, je nach Erfordernis oder Wunsch der die Testperson behandelnden Person, der Messfühler manuell zwischen den zwei plattenförmigen Bauteilen bewegbar oder starr zwischen den zwei plattenförmigen Bauteilen angeordnet sein.

Um eine möglichst genaue Messung des zu untersuchenden Organs der Testperson zu gewährleisten, wird das Messelement vorzugsweise mit einer Hand einer Testperson in Kontakt gebracht, da bekanntermaßen die Fingerspitzen des Menschen auf Grund ihrer Empfindlichkeit und wegen der Vielfalt der Messpunkte an den Meridianen am besten zur Messung der Frequenz geeignet sind.

Liegt demnach der von dem am Körper der Testperson angelegten Messelement übermittelte Frequenzwert in dem von der rechnergesteuerten Auswerteeinheit vorgegebenen Frequenzbereich des zu untersuchende Organs, der ja bekannt ist, wird die Antenne zwischen den plattenförmigen Bauteilen in vertikaler Richtung ausschlagen. Dies ist ein Zeichen dafür, dass das zu untersuchende Organ der Testperson gesund ist. Wenn jedoch der vom Messelement am Körper der Testperson übermittelte Frequenzwert außerhalb des von der rechnergesteuerten Auswerteeinheit vorgegebenen Frequenzbereiches oder Frequenzwertes liegt, schlägt die Antenne in horizontaler Richtung zwischen den plattenförmigen Bauteilen aus. Das bedeutet, dass das zu untersuchende Organ der Testperson erkrankt ist.

Um die Messung der Frequenz an den am Körper bzw. an den Messpunkten der Meridiane eines Fingers der Hand der Testperson überhaupt zu ermöglichen und das Messergebnis auf das zugeordnete plattenförmige Bauteil übertragen zu können, besteht das mit dem Körper einer Testperson in Kontakt bringbare Messelement aus einem elektrisch leitenden Material.

Zur wechselseitigen Übertragung bzw. Vorgabe von Messwerten sind das mit dem Körper einer Testperson in Kontakt bringbare Messelement über eine elektrische Leitung mit einem der plattenförmigen Bauteile und das andere plattenförmige Bauteil über eine elektrische Leitung mit der rechnergesteuerten Auswerteeinheit verbunden. Zweckmäßigerweise ist die rechnergesteuerte Auswerteeinheit eine elektronische Datenverarbeitungseinrichtung. Weiterhin kann der Messfühler über elektrische Leitungen mit jedem der plattenförmigen Bauteil verbunden sein. Bevorzugt weist hierfür jedes der plattenförmige Bauteile hierzu Anschlussbuchsen für die elektrischen Leitungen auf.

Andererseits kann auf die elektrischen Leitungen vom Messfühler zu den plattenförmigen Bauteilen verzichtet werden. Da sich zwischen den plattenförmigen Bauteilen ein elektromagnetisches Feld bildet, wenn die Auswerteeinheit eingeschaltet ist und dadurch die Diagnostiziereinrichtung mit Spannung versorgt, wird bei Kontakt des Messelementes mit der Testperson von der Antenne des Messfühlers die Frequenz des elektromagnetischen Feldes gemessen und die gemessenen Werte an die Auswerteeinheit übertragen.

Nach einer Weiterbildung der erfindungsgemäßen Diagnostiziereinrichtung ist die Auswerteeinheit mit einer Therapieeinheit verbunden. Die Auswerteeinheit wertet die von an der Testperson ermittelten Messwerte aus und ermittelt dann gleichzeitig durch ein internes Computerprogramm Vorgabewerte für eine nachfolgende Therapie der Testperson, d.h. der nun zu therapierenden Person. Die Vorgabewerte werden an die Therapieeinheit übertragen und bestimmen den Therapieverlauf an der zu therapierenden Person.

Durch ein zusätzliches Computerprogramm oder ein in das Diagnoseprogramm integriertes Computerteilprogramm in der Auswerteeinheit der Diagnostiziereinrichtung werden somit die gemessenen und ausgewerteten Messwerte und die daraus ermittelten Vorgabewerte für die Therapie der zu therapierenden Person an die Therapieeinheit übermittelt, welches die Therapie an der Person vornimmt.

Ferner ist die Therapieeinheit in Kontakt mit einer zu therapierenden Person bringbar. Dabei werden Elektroden an das zu therapierende Körperteil der Person angebracht und aus den Vorgabewerten resultierende Spannungsfrequenzen auf die zu therapierende Person übertragen.

Häufig liegt zwischen Diagnose und Therapie ein mehrtägiger Zeitraum oder es genügt nicht nur eine einmalige Therapiesitzung an der zu therapierenden Person, so dass zwischen den Sitzungen bestimmte Zeitspannen liegen. Um diese Zeiträume therapeutisch zu überbrücken, kann sonach die Auswerteeinheit Software gestützt bestimmte Medikamente, die auf die zu therapierende Person im Hinblick auf deren Krankheitsbild spezifisch zugeschnitten sind, ermitteln, welche die Person in diesen Zeiträumen einnehmen soll. Nach jeder Therapiesitzung sollte eine erneute Diagnose mit der Diagnostiziereinrichtung vorgenommen werden, da sich die ursprünglichen Messwerte infolge des Zeitversatzes in der Regel geändert haben.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Der der Erfindung zu Grunde liegende Gedanke wird in der nachfolgenden Beschreibung anhand eines Ausführungsbeispieles, das in der Zeichnung dargestellt ist, näher beschrieben. Es zeigen:
- Fig. 1: eine schematische Darstellung der erfindungsgemäßen Diagnostiziereinrichtung in einer Draufsicht mit Anschluss an eine Auswerteeinheit und eine Therapieeinheit
- Fig. 2: eine schematische Seitenansicht der Diagnostiziereinrichtung nach Fig. 1.

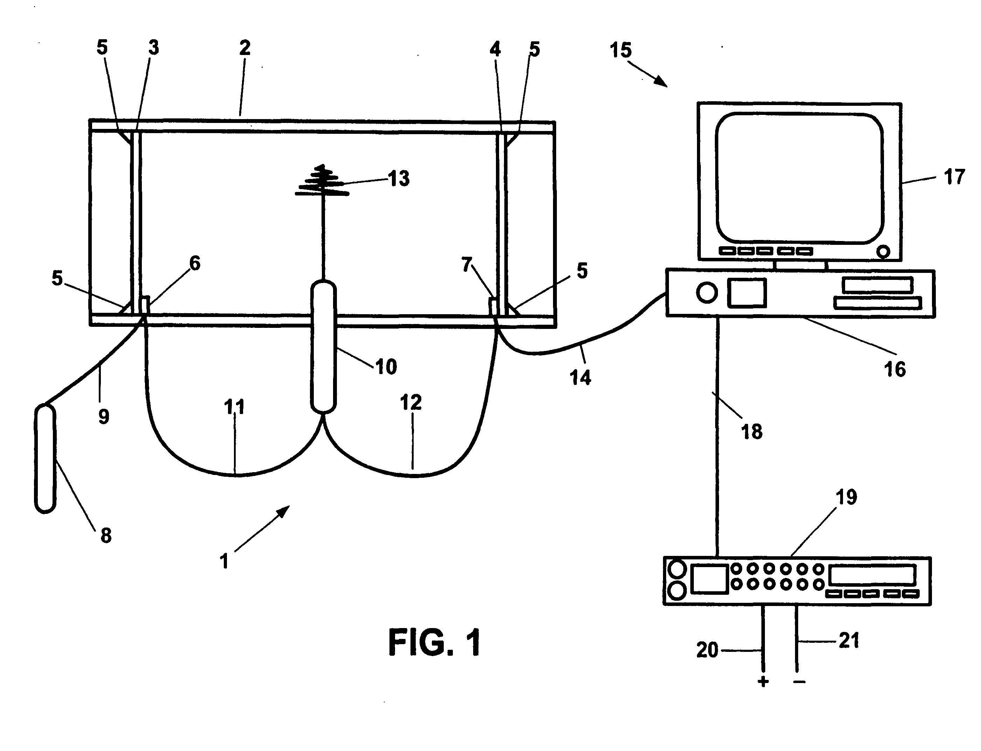

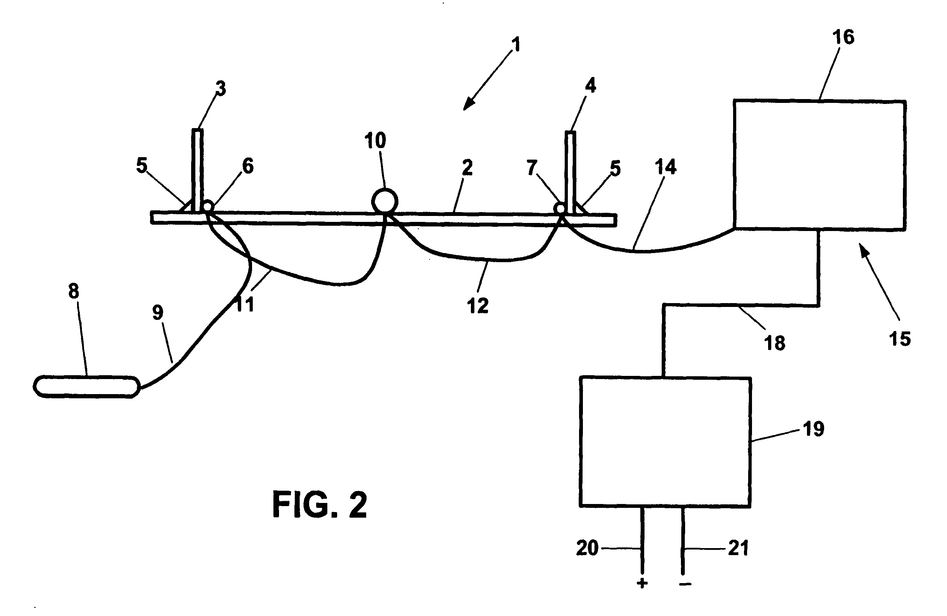

Die Diagnostiziereinrichtung 1 gemäß den Fig. 1 und 2 weist eine Basisplatte 2 auf. Auf der Basisplatte 2 sind in einem bestimmten Abstand zueinander zwei plattenförmige Bauteile 3 und 4 parallel zueinander angeordnet, wobei diese lotrecht von Basisplatte 2 hervorragen, wie das insbesondere aus Fig. 2 ersichtlich ist. Jedes der plattenförmigen Bauteile 3 und 4 ist mit Plattenhaltern 5 oder anderen geeigneten Befestigungsmitteln an der Basisplatte 2 befestigt. Dadurch ist gewährleistet, dass die plattenförmige Bauteil 3 und 4 immer einen gleichen Abstand zueinander aufweisen. Des Weiteren umfasst jedes dieser plattenförmigen Bauteile 3 und 4 eine Anschlussbuchse 6 bzw. 7 zur Aufnahme von elektrischen Leitungen.

Zur Diagnostiziereinrichtung 1 gehört weiterhin ein handgriffartiges Messelement 8 aus einem elektrisch leitfähigen Material. Dieses Messelement 8 ist über eine elektrische Leitung 9 mit der Anschlussbuchse 6 des plattenförmigen Bauteils 3 verbunden. Des Weiteren umfasst die Diagnostiziereinrichtung 1 einen handgriffartigen Messfühler 10, der einerseits über eine elektrische Leitung 11 mit der Anschlussbuchse 6 des plattenförmigen Bauteils 3 und andererseits über eine elektrische Leitung 12 mit der Anschlussbuchse 7 des plattenförmigen Bauteils 4 verbunden ist. Der Messfühler 10 weist eine Antenne 13 auf, deren vorderes Ende spiralförmig ausgebildet ist und die aus einem elektrisch leitenden Metalldraht besteht.

Die Diagnostiziereinrichtung 1 ist über eine elektrische Leitung 14 mit einer rechnergesteuerten Auswerteeinheit 15 verbunden. Diese Auswerteeinheit 15 besteht aus einem Computer 16 und einem Bildschirm 17. Im Computer 16 ist ein spezielles Computerprogramm enthalten, welches bestimmte bekannte Frequenzinformationen über die Leitung 14 an das plattenförmige Bauteil 4 abgibt.

Das Messelement 8 kontaktiert die Hand bzw. den Finger einer Testperson, die auf Erkrankung oder Nichterkrankung eines bestimmten Organs untersucht werden soll. Durch das Messelement 8 wird die Frequenz der Haut bzw. der Gewebeschichten von bestimmten Messpunkten eines Meridians, der für ein bestimmtes Organ zuständig ist, über die Leitung 9 zum plattenförmigen Bauteil 3 übertragen. Gleichzeitig erfolgt die Vorgabe eines bestimmten Frequenzbereiches oder Frequenzwertes für das zu untersuchende Organ der Testperson von der rechnergesteuerten Auswerteeinheit 15 zum plattenförmigen Bauteil 4. Dadurch wird ein elektrisches Feld zwischen den plattenförmigen Bauteilen 3 und 4 erzeugt. Mit Hilfe des handgriffartigen Messfühlers 10 und dessen Antenne 13, der von einer untersuchenden Person gehandhabt wird, wird ein möglicher Unterschied in der Feldcharakteristik zwischen den beiden plattenförmigen Bauteilen 3 und 4 gemessen und über die Leitungen 12 und 14 an die rechnergesteuerte Auswerteeinheit 15 übertragen. Der Computer 16 der Auswerteeinheit 15 erstellt mittels Computerprogramm aus den erhaltenen Informationen einen Therapieplan oder einen Plan zur Verabreichung von Medikamenten zur Behandlung des erkrankten Organs der Testperson.

Die Auswerteinheit 15 ist über eine Leitung 18 mit einer Therapieeinheit 19 verbunden. Dabei übermittelt die Auswerteinheit 15 die vom Computerprogramm ermittelten Vorgabewerte für eine Therapie des bestimmten Körperteils der zu therapierenden Person an die Therapieeinheit 19. Über von der Therapieeinheit 19 ausgehende Kontakte 20 und 21, die am zu therapierenden Körperteil der zu therapierenden Person angebracht werden, werden entsprechende Spannungsfrequenzen auf der Basis der Vorgabewerte der Auswerteeinheit 15 von der Therapieeinheit 1 zum zu therapierenden Körperteil der zu therapierenden Person übertragen.

### Liste der Bezugszeichen

- 1: Diagnostiziereinrichtung
- 2: Basisplatte
- 3: plattenförmiges Bauteil
- 4: plattenförmiges Bauteil
- 5: Plattenhalter
- 6: Anschlussbuchse
- 7: Anschlussbuchse
- 8: Messelement
- 9: Leitung
- 10: Messfühler
- 11: Leitung
- 12: Leitung
- 13: Antenne
- 14: Leitung
- 15: Auswerteeinheit
- 16: Computer
- 17: Bildschirm
- 18: Leitung
- 19: Therapieeinheit

## Patentansprüche

1. Diagnostiziereinrichtung zum Testen von Organen des Menschen durch Messung der Stromfrequenz des zu testenden Organs, **gekennzeichnet durch** zwei parallel zueinander beabstandete, vertikal aufgerichtete plattenförmige Bauteile (3, 4), die mit einem zwischen diesen einbringbaren Messfühler (10) elektrisch verbunden sind, wobei eines der plattenförmigen Bauteile (3) mit einem mit dem Körper einer Testperson in Kontakt bringbaren Messelement (8) und das andere plattenförmige Bauteil (4) mit einer rechnergesteuerten Auswerteeinheit (15) elektrisch verbunden sind.

2. Diagnostiziereinrichtung nach Anspruch 1, **dadurch gekennzeichnet, dass** die plattenförmigen Bauteile (3, 4) aus einem elektrisch leitenden Material bestehen.

3. Diagnostiziereinrichtung nach Anspruch 1 oder 2, **dadurch gekennzeichnet, dass** die plattenförmigen Bauteile (3, 4) auf einem Basisträger (2) befestigt sind.

4. Diagnostiziereinrichtung nach einem der Ansprüche 1 bis 3, **dadurch gekennzeichnet, dass** der Messfühler (10) als Antenne (13) ausgebildet und zur Stromfrequenzmessung zwischen den zwei plattenförmigen Bauteilen (3, 4) vorgesehen und manuell zwischen den zwei plattenförmigen Bauteilen (3, 4) bewegbar oder starr zwischen den zwei plattenförmigen Bauteilen (3, 4) angeordnet ist.

5. Diagnostiziereinrichtung nach einem der Ansprüche 1 bis 4, **dadurch gekennzeichnet, dass** das Messelement (8) vorzugsweise mit einer Hand einer Testperson in Kontakt bringbar ist.

6. Diagnostiziereinrichtung nach einem der Ansprüche 1 bis 5, **dadurch gekennzeichnet, dass** das mit dem Körper einer Testperson in Kontakt bringbare Messelement (8) aus einem elektrisch leitenden Material besteht.

7. Diagnostiziereinrichtung nach einem der Ansprüche 1 bis 6, **dadurch gekennzeichnet, dass** das mit dem Körper einer Testperson in Kontakt bringbare Messelement (8) über eine elektrische Leitung (9) mit einem der plattenförmigen Bauteile (3) und das andere plattenförmige Bauteil (4) über eine elektrische Leitung (14) mit der rechnergesteuerten Auswerteeinheit (15) verbunden sind.

8. Diagnostiziereinrichtung nach einem der Ansprüche 1 bis 7, **dadurch gekennzeichnet, dass** der Messfühler (10) über elektrische Leitungen (11, 12) mit jedem der plattenförmigen Bauteil (3, 4) verbunden ist.

9. Diagnostiziereinrichtung nach einem der Ansprüche 1 bis 8, **dadurch gekennzeichnet, dass** die Auswerteeinheit (15) mit einer Therapieeinheit (19) verbunden ist.

10. Diagnostiziereinrichtung nach Anspruch 9, **dadurch gekennzeichnet, dass** die Therapieeinheit (19) in Kontakt mit einer zu therapierenden Person bringbar ist.

## Claims

1. Diagnostic device for the testing of human organs by measuring the current frequency of the organ under examination, **characterised by** two vertically arranged planar components(3, 4), set up in parallel to each other, which are electronically connected to a measuring sensor (10) that can be moved between them, one of the planar components (3) being connected to a measuring element (8) that can be brought into contact with the body of the test person, and the other planar component (4) being electronically connected to a computer-controlled evaluation unit (15).

2. Diagnostic device in accordance with claim 1, **characterised by** the fact that the planar components (3, 4) consist of an electrically conductive material.

3. Diagnostic device in accordance with claim 1 or 2, **characterised by** the fact that the planar components (3, 4) are attached to a base plate (2).

4. Diagnostic device in accordance with claims 1 to 3, **characterised by** the fact that the measuring sensor (10) is designed as an antenna (13) and allows the measurement of the current frequency between the two planar components (3, 4) and can either be moved manually between the two planar components (3, 4) or placed in a fixed position between the two planar components (3, 4).

5. Diagnostic device in accordance with claims 1 to 4, **characterised by** the fact that the measuring element (8) can preferably be brought into contact with one of the hands of the test person.

6. Diagnostic device in accordance with claims 1 to 5, **characterised by** the fact that the measuring element (8) that can be brought into contact with the body of the test person consists of an electrically conductive material.

7. Diagnostic device in accordance with claims 1 to 6, **characterised by** the fact that the measuring element (8) that can be brought into contact with the body of the test person is connected via an electrical lead (9) to one of the planar components (3) and the other planar component (4) is connected via an electrical lead (14) to the computer-controlled evaluation unit (15).

8. Diagnostic device in accordance with claims 1 to 7, **characterised by** the fact that the measuring sensor (10) is connected via electrical leads (11, 12) to both of the planar components (3, 4).

9. Diagnostic device in accordance with claims 1 to 8, **characterised by** the fact that the evaluation unit (15) is connected to a therapy unit (19).

10. Diagnostic device in accordance with claim 9, **characterised by** the fact that the therapy unit (19) can be brought into contact with a person undergoing therapy.

## Revendications

1. Dispositif de diagnostic pour tester les organes humains par mesure de la fréquence électrique de l'organe à tester **caractérisé par** deux éléments en forme de plaque (3, 4) placés verticalement éloignés parallèlement l'un par rapport à l'autre qui sont reliés électriquement à un détecteur de mesure (10) pouvant être installé entre les deux éléments, l'un des éléments en forme de plaque (3) étant relié électriquement à un élément de mesure (8) pouvant être mis en contact avec le corps d'une personne-test et l'autre élément en forme de plaque (4) à une unité d'analyse (15) commandée par ordinateur.

2. Dispositif de diagnostic selon la revendication 1, **caractérisé en ce que** les éléments en forme de plaque (3, 4) se composent d'un matériau à conduction électrique.

3. Dispositif de diagnostic selon la revendication 1 ou 2, **caractérisé en ce que** les éléments en forme de plaque (3, 4) sont fixés sur un support de base (2).

4. Dispositif de diagnostic selon l'une des revendications 1 à 3, **caractérisé en ce que** le détecteur de mesure (10) est formé comme antenne (13) et qu'il est prévu pour mesurer la fréquence électrique entre les deux éléments en forme de plaque (3, 4) et placé de façon mobile entre les deux éléments en forme de plaque (3, 4) ou de façon fixe entre les deux éléments en forme de plaque (3, 4).

5. Dispositif de diagnostic selon l'une des revendications 1 à 4, **caractérisé en ce que** l'élément de mesure (8) peut être mis en contact de préférence avec une main d'une personne-test.

6. Dispositif de diagnostic selon l'une des revendications 1 à 5, **caractérisé en ce que** l'élément de mesure (8) pouvant être mis en contact avec le corps d'une personne-test se compose d'un matériau à conduction électrique.

7. Dispositif de diagnostic selon l'une des revendications 1 à 6, **caractérisé en ce que** l'élément de mesure (8) pouvant être mis en contact avec le corps d'une personne-test est relié à l'un des éléments en forme de plaque (3) par un câble électrique (9) et l'autre élément en forme de plaque (4) à l'unité d'analyse commandée par ordinateur (15) par un câble électrique (14).

8. Dispositif de diagnostic selon l'une des revendications 1 à 7, **caractérisé en ce que** le détecteur de mesure (10) est relié à chacun des éléments en forme de plaque (3, 4) par câbles électriques (11, 12).

9. Dispositif de diagnostic selon l'une des revendications 1 à 8, **caractérisé en ce que** l'unité d'analyse (15) est reliée à une unité de thérapie (19).

10. Dispositif de diagnostic selon la revendication 9, **caractérisé en ce que** l'unité de thérapie (19) peut être mise en contact avec une personne devant subir une thérapie.
